## Europäisches Patentamt
## European Patent Office
## Office européen des brevets

(19)

(11) Numéro de publication: **0 188 945**
**B1**

(12) # FASCICULE DE BREVET EUROPÉEN

(45) Date de publication du fascicule du brevet:
**12.10.88**

(51) Int. Cl.⁴: **C 07 C 53/16,** C 07 C 51/363

(21) Numéro de dépôt: **85402510.3**

(22) Date de dépôt: **16.12.85**

(54) **Procédé de préparation d'acide monochloracétique à partir d'acide glycolique.**

(30) Priorité: **20.12.84 FR 8419554**

(43) Date de publication de la demande:
**30.07.86 Bulletin 86/31**

(45) Mention de la délivrance du brevet:
**12.10.88 Bulletin 88/41**

(84) Etats contractants désignés:
**AT BE CH DE FR GB IT LI LU NL SE**

(56) Documents cité:
**GB-A-727 074**
**US-A-2 876 255**

(73) Titulaire: **ATOCHEM, 4 & 8, Cours Michelet La
Défense 10, F-92800 Puteaux (FR)**

(72) Inventeur: **Correia, Yves, Les Lauzières, F-04160
Château- Arnoux (FR)**
Inventeur: **Vermont, Jean, 9, Rue Languedoc,
F-04600 Saint- Auban (FR)**

(74) Mandataire: **Rochet, Michel, ATOCHEM
Département Propriété Industrielle, F-92091 Paris
la Défense Cédex 42 (FR)**

EP 0 188 945 B1

# 0 188 945

## Description

La présente invention concerne un procédé de préparation d'acide monochloracétique à partir d'acide glycolique.

L'acide monochloracétique est de plus en plus préparé à l'échelle industrielle à partir d'acide acétique même si une telle méthode conduit à la formation d'acide dichloracétique, difficilement séparable de l'acide monochloracétique par les techniques habituelles de distillation. C'est notamment dans le but de résouers ce problème que le brevet américain 422 1921 propose de préparer l'aciée monochloracétique à partir d'acide glycolique. Selon ce brevet, la réaction est effectuée entre 100 et 250°C, en présence d'acide iodhydrique. La voie d'accés à l'acide monochloracétique à partir d'acide glycolique apparaît prometteuse dans la mesure où les nouveaux développements de la préparation de cet acide glycolique, notamment par carbonylation du formaldéhyde laissent espérer une baisse sensible du prix de revient du dit acide glycolique.

Le brevet américain 2 876 255 enseigne l'hydrobromation de l'acide glycolique, la réction peut se faire en présence de certains catalysens identiques à ceux de la présente invention. Le brevet explique en colonne 2, lignes 9 - 55 qu'il est nécessaire d'avoin un milieu réactionnel le plus anhydre possible et qu'au moins il faut éviter l'accumulation d'eau qui se forme lors de la réaction du groupe OH sur HBr. C'est pourquoi la réaction est, dans tous les exemples, effectuée dans un appareil comprenant une colonne à distiller. Or, dans la présente invention, les conditions opératoires sont sans importance, les exemples sont réalisés en ampoules scellées, c'est-à-dire que l'eau de réactiom me peut s'échapper.

La présente invention a donc pour objet un procédé de préparation d'acide monochloracétique par hydrochloration de l'acide glycolique, ce procédé étant caractérisé en ce que la réaction est effectuée en présence d'un catalyseur constitué par un acide de Lewis autre qu'un hydracide.

L'invention concerne tout particulièrement un procédé dans lequel l'acide de Lewis est mis en oeuvre sous forme libre ou fixé sur un support solide.

Elle vise plus précisément un procédé de fabrication d'acide monochloracétique par hydrochloration d'acide glycolique, le dit procédé étant caractérisé en ce que la réaction est effectuée en présence d'un ou plusieurs catalyseurs choisis dans le groupe constitue par les halogénures métalliques ou métalloïdiques, les acides minéraux oxygénés et les acides carboxyliques ou sulfoniques.

A titre d'illustration des halogénures métalliques ou métalloïdiques, on citera tout particulièrement $BF_3$, mis en oeuvre tel quel ou sous forme de complexe $HF/BF_3$, $BCl_3$, $ZnCl_2$, $FeCl_3$, $AlCl_3$, $SbCl_3$.

A titre d'illustration des acides minéraux oxygénés, on citera notamment $H_2SO_4$ et $H_3PO_4$.

A titre d'illustration des acides carboxyliques ou sulfoniques, on citera notamment les acides de formules R COOH et R $SO_3H$ dans lesquelles R désigne un radical aliphatique renfermant de 1 à 6 atomes de carbone, non substitué ou substitué par un ou plusieurs atomes d'halogène et notamment de fluor, ou un radical comportant un noyau aromatique et 6 à 12 atomes de carbone. Parmi de tels acides on citera notamment l'acide trifluoroacétique, l'acide trifluorométhane sulfonique, l'acide benzène sulfonique et les acides toluéne-sulfoniques.

Dans la mise en oeuvre du procédé conforme à l'invention, l'acide chlorhydrique utilisé pour la réaction d'hydrochloration de l'acide glycolique en acide monochloracétique peut être sous forme anhydre ou sous forme de solution aqueuse pouvant renfermer jusqu'à 90 % en poids d'eau. D'une manière générale, on préfère utiliser de l'acide chlorhydrique aqueux, à des concentrations pouvant être comprises entre 20 et 70 % en poids d'HCl par rapport au poids de la solution.

L'acide chlorhydrique est utilisé généralement dans un rapport molaire $\frac{HCL}{acide\ glycolique}$ compris entre 20/1 et 1/20, et de préférence compris entre 10/1 et 1/5. On préfère cependant opérer avec un excès molaire d'HCl et plus précisément un rapport molaire $\frac{HCl}{acide\ glycolique}$ compris entre 1,1/1 et 5/1.

La réaction d'hydrochloration peut être effectuée entre 80 et 200°C. De préférence la température est comprise entre 100 et 150°C.

La réaction peut s'effectuer à pression atmosphérique ou sous une pression absolue pouvant atteindre 30 bars. De préférence on opère sous une pression absolue comprise entre 2 et 10 bars. La durée de la réaction est dépendante du taux de conversion d'acide glycolique en acide monochloracétique désiré. De ce fait la durée de réaction peut aller de quelques heures, 3 par exemple à 1 jour, voire davantage.

Le rapport molaire catalyseur/acide glycolique est généralement compris entre 1/1000 et 10/1 et, de préférence, entre 2/100 et 1/10.

Le procédé conforme à l'invention permet un accés facile à l'acide monochloracétique à partir d'acide glycolique avec d'excellents taux de conversion, l'acide monochloracétique pouvant facilement être séparé de l'acide glycolique par distillation.

Les exemples suivants illustrent l'invention.

## EXEMPLES

On chauffe à 130°C une ampoule en pyrex scellée contenant:
<u>Exemple 1</u>
- 15,6 g d'acide glycolique (0,205 mole)

2

- 50 cm³ de solution aqueuse d'HCl à 37,9 % (0,615 mole)
- 0,873 g de ZnCl₂ (0,0064 mole)

Exemple 2

- 15,6 d'acide glycolique
- 50 cm³ de la solution d'HCl de l'exemple 1
- 0,628 g d'acide sulfurique (0,0064 mole)

Exemple 3

- 15,6 g d'acide glycolique
- 50 cm³ de la solution d'HCl de l'exemple 1
- 0,73 g d'acide trifluoroacétique (0,0064 mole)

Exemple a (témoin)

- 15,6 g d'acide glycolique
- 50 cm³ de la solution d'HCl de l'exemple 1
- pas de catalyseur

Exemple b (témoin)

- 15,6 g d'acide glycolique
- 50 cm³ de la solution d'HCl de l'exemple 1
- 0,818 g d'acide iodhydrique (0,0064 mole)

Au bout de 4, 8 ou 16 heures, on détermine par mesure de la consommation d'ions chlorure de la solution, un taux de transformation d'acide glycolique en acide monochloracétique.

Ces résultats sont rassemblés dans le tableau qui suit:

| Exemple | Catalyseur | Taux de conversion après | | |
|---------|------------|----------|----------|-----------|
| | | 4 heures | 8 heures | 16 heures |
| 1 | $ZnCl_2$ | 16,4 | 21,4 | 28 |
| 2 | $H_2SO_4$ | 11,2 | 25,4 | 40,8 |
| 3 | $CF_3COOH$ | 14,6 | 23,7 | 46,5 |
| Témoins | | | | |
| a | - | 10,6 | 23,8 | 33,9 |
| b | HI | 14,7 | 17 | 30 |

Dans tous les cas la sélectivité en acide monochloracétique est de 100 %. Il ressort de ce tableau:

- que $ZnCl_2$ est tout à fait comparable à la catalyse par HI au bout de 16 heures tout en ayant au départ (4 heures) une cinétique initiale plus rapide.

- que $H_2SO_4$ et $CF_3COOH$ permettent d'obtenir, dès 130°C des taux de transformation très élevés après 16 heures de réaction.

**Revendications**

1. Procédé de préparation d'acide monochloracétique par hydrochloration de l'acide glycolique, ce procédé étant caractérisé en ce que la réaction est effectuée en présence d'un catalyseur constitué par un acide de Lewis autre qu'un hydracide.

2. Procédé selon la revendication 1, caractérisé en ce que la réaction est effectuée en présence d'un ou plusieurs catalyseurs choisis dans le groupe constitué par les halogénures métalliques ou métalloïdiques, les acides minéraux oxygénés et les acides carboxyliques ou sulfoniques.

3. Procédé selon l'une quelconque des revendications 1 ou 2, caractérisé en ce que l'acide chlorhydrique utilisé pour la réaction d'hydrochloration d'acide glycolique en acide monochloracétique est sous forme anhydre ou sous forme de solution aqueuse pouvant renfermer jusqu'à 90 % en poids d'eau.

4. Procédé selon l'une quelconque des revendications 1 à 3, caractérisé en ce que le rapport molaire $\frac{HCl}{acide\ glycolique}$ est compris entre 20/1 et 1/20 et de préférence entre 10/1 et 1/5.

5. Procédé selon l'une quelconque des revendications 1 à 3, caractérisé en ce que la rapport molaire $\frac{HCl}{acide\ glycolique}$ est compris entre 1,1/1 et 5/1.

6. Procédé selon l'une quelconque des revendications 1 à 5, caractérisé en ce que le rapport molaire $\frac{catalyseur}{acide\ glycolique}$ est compris entre 1/1000 et 10/1 et, de préférence, entre 2/100 et 1/10.

**Patentansprüche**

1. Verfahren zur Herstellung von Monochloressigsäure durch Hydrochloricrung von Glykolsäure, dadurch gekennzeichnet, daß die Reaktion in Anwesenheit eines Katalysators durchgeführt wird, der eine Lewis-Säure anderer Art, als eine Wasserstoffsäure ist.

2. Verfahren nach Patentanspruch 1, dadurch gekennzeichnet, daß die Reaktion in Anwesenheit eines oder mehrerer Katalysatoren ausgewählt aus der Gruppe bestehend aus den Metall- oder Halbmetallhalogeniden, den sauerstoffhaltigen Mineralsäuren und den Carbonsäuren oder Sulfonsäuren durchgeführt wird.

3. Verfahren nach irgendeinem der Patentansprüche 1 oder 2, dadurch gekennzeichnet, daß die zur Hydrochlorierung der Glykolsäure zur Monochloressigsäure verwendete Salzsäure als Anhydrid oder als wässrige Lösung mit bis zu 90 Gewichtsprozent Wasser angewendet wird.

4. Verfahren nach irgendeinem der Patentansprüche 1 bis 3, dadurch gekennzeichnet, daß das Molverhältnis HCl/Glykolsäure zwischen 20/1 und 1/20 und vorzugsweise zwischen 10/1 und 1/5 liegt.

5. Verfahren nach irgendeinem der Patentansprüche 1 bis 3, dadurch gekennzeichnet, daß das Molverhältnis HCl/Glykolsäure zwischen 1,1/1 und 5/l liegt.

6. Verfahren nach irgendeinem der Patentansprüche 1 bis 5, dadurch gekennzeichnet, daß das Molverhältnis Katalysator/Glykolsäure zwischen 1/1000 und 10/1 und vorzugsweise zwischen 2/100 und 1/10 liegt.

**Claims**

1. Process for the preparation of monochloroacetic acid by hydrochlorination of glycolic acid, this process being characterised in that the reaction is carried out in the presence of a catalyst consisting of a Lewis acid other than a hydracid.

2. Process according to Claim 1, characterised in that the reaction is carried out in the presence of one or more catalysts chosen from the group consisting of metal or metalloid halides, oxygen-containing inorganic acids and carboxylic or sulphonic acids.

3. Process according to either of Claims 1 or 2, characterised in that the hydrochloric acid employed for the hydrochlorination reaction of glycolic acid to monochloroacetic acid is in anhydrous form or in the form of an aqueous solution which may contain up to 90 % by weight of water.

4. Process according to any one of Claims 1 to 3, characterised in that the HCl/glycolic acid molar ratio is from 20/1 to 1/20 and preferably from 10/1 to 1/5.

5. Process according to any one of Claims 1 to 3, characterised in that the HCl/glycolic acid molar ratio is from 1.1/1 to 5/1.

6. Process according to any one of Claims 1 to 5, characterised in that the molar ratio catalyst/glycolic acid is from 1/1000 to 10/1 and, preferably, from 2/100 to 1/10.